# EUROPEAN PATENT SPECIFICATION

(11) **EP 0 769 303 B1**
(45) Date of publication and mention of the grant of the patent: **30.07.2003**
(21) Application number: 96116050.4
(22) Date of filing: 07.10.1996
(51) Int. Cl.: A61M 15/00, A61B 5/087

(54) **Combined inhaler and peak flow meter**
Kombinierter Inhalator und Spitzendurchflussmessgerät
Inhalateur combiné avec un débitmètre de crête

(30) Priority: 12.10.1995 IT MI952078
(43) Date of publication of application: 23.04.1997
(73) Proprietor: SHIRE ITALIA S.P.A., 35127 Padova (IT)
(72) Inventor: Frati, Franco, 52144 Cortona, Arezzo (IT); Albiani, Claudio, 52045 Foiano Della Chiana, Arezzo (IT)
(74) Representative: Gervasi, Gemma, Dr.

(56) References cited:
- EP-A- 0 406 893
- WO-A-90/09203
- DE-A- 3 040 641
- GB-A- 2 279 879
- US-A- 3 635 214
- US-A- 3 732 864
- US-A- 3 870 046

## Description

### Field of the Invention

The present invention relates to a device for the treatment of asthmatic patients, suitable for performing the double function of measuring the peak expiratory flow rate (hereinafter referred to as "PEFR") and of delivering a drug for the treatment of asthma.

### Prior Art

The asthmatic syndromes of any type (intrinsic and extrinsic, etc.) are a very important socio-sanitary problem: the progressive increase in the air pollution and the constant increase in the number of subjects with allergic reactions reasonbly allow to believe that the number of asthmatic patients and/or the importance of the asthmatic manifestations will increase, maybe even in a considerable manner.

Although new and more efficient drugs for the treatment of the asthmatic manifestations and new diagnostic methods for determining the presence and severity of asthma are available which enable the physician to prescribe the best treatment to each patient, by deciding the treatment and the dosage each time in the best manner, there hasn't been much to do to obviate what many researchers consider the main problem in the treatment of asthmatic patients: the risk for the patient to use the drug in an incongruous manner, by taking it at too high doses and/or in case of no real need, not respecting the dosage prescribed by the physician.

Such behaviour exposes the patient to the danger of drug poisonings (often resulting from and/or made worse by the synergic action of two or more drugs taken without previous medical prescription and/or medical control) which can be dangerous for the health of the patient and even fatal.

Some researchers and clinicians think that the risks relative to the treatment of asthmatic patients are due to an incrongruous use of drugs ("beta 2 agonists", particularly) which, not having antiinflammatory action, but bronchodilatation action, only, are unable to interact with pulmonary inflammation (important morphologic condition relative to asthma), exposing the patient to severe consequences.

In the medical literature many studies are available which prove how a careful monitoring of the parameters of lung function can improve quality of life of asthmatic patients, first of all by allowing a more rational use of the different drugs in question.

Asthmatic patients haven't generally the opportunity (nor have they often the possibility) to have recourse to sanitary personnel and/or to sanitary structures to perform such monitoring, particularly in case of exacerbations: monitoring performed by the patient at home by means of meters easy to use (such as peak flow meter) would allow the patient himself to take drugs in question in case of real need, only, and according to the dosage prescribed by the physician (dosage which is or can be related to the severity of asthma), such monitoring significantly helping the "self-management" of the asthmatic syndrome performed by the patient himself, which is considered of great importance by the most part of those skilled in the art.

Attempts up to now performed to carry out campaigns for monitoring asthma at home by means of measurement of PEFR at first showed encouraging results, but the compliance of the patient progressively decreased, which was a serious problem relative to the adoption of such method to a vast extent.

It is believed that the scant compliance of the patients is due to the fact that the patient, being always obliged to carry with himself at least one package of drug and the respective device for delivering the drug, considers the peak flow meter cumbersome, he doesn't use it in case of need and at last he won't bring it with himself anymore, at the expense of the quality of the management of asthma.

The device subject-matter of the present invention allows to obviate said problem, helping the patient in the management of asthma as it contains in a sole (not very cumbersome) container means by which it can perform the double function of peak flow meter and of device for delivering the drug used in the treatment of asthma.

The fact that both functions (measurement of PEFR and delivery of the drug) are always at the patient's disposal and that the patient can easily select one function or the other one is advantageous as it allows the patient a better management of asthma: as a matter of fact, a timely measurement of PEFR succeeds in calming an anxious patient, allowing to avoid (if the measured PEFR belongs to the normal range) the intake of drug in case of no real need, whereas, in case of asthma attack, the measurement itself can give the patient information relative to the dosage of drug to be taken to control the attack.

The physician, in fact, can determine (for each patient) PEFR values which can be considered normal for the patient and PEFR values which suggest the intake of one or more doses of drug; such "personalized" information can be written on stick-on labels to be applied onto the external surface of the present invention so that they are always at the patient's disposal.

Moreover, the present invention allows (the physician and/or the patient) to discriminate easily if symptoms the patient complains of (e.g. a chronic cough), hardly attributable to asthma, are (or can be) related to asthma or if they must be attributed to a different pathology.

To perform "screening" it's sufficient that the patient measures his PEFR: if the value belongs to the normal range, symptoms the patient complains of can be attributed to a pathology different from asthma and can be treated with specific drugs, otherwise, if the PEFR value doesn't belong to the normal range, the patient will take a dose of anti-asthma drug and will repeat the measurement of his PEFR: if the new value belongs to the normal range (or it has neared such range), the symptoms the patient complains of can be attributed to asthma and treated accordingly, otherwise, if the new value is almost the same as the previous one, symptoms the patient complains of can be attributed to pathology different from asthma and treated with specific drugs.

Therefore, a better management of asthma by the patient is particularly advantageous by using a device according to the present invention, a not very cumbersome and easy to use device, suitable for performing the double function of peak flow meter and of device for the delivery of drugs used in the treatment of asthma.

US-A-3 635 214 refers to a peak flow meter which is also convertible for use as medicinal inhalator through a breathing tube which may include an adapter for a container delivering drugs used in the treatment of asthma; it provides therefore for a double-purpose breathing tube suitable to be used either by the peak flow meter or by the drugs delivery container.

GB-A-2 279 879 refers to a medicament inhaler comprising a generally frusto-conical chamber positioned between the mouthpiece and the housing for receiving a pressurised dispensing container, the section of said chamber being increasing from the housing to the mouthpiece.

### Summary of the Invention

The present invention relates to a device for the treatment of asthmatic patients suitable for performing the double function of measuring the peak expiratory flow rate (PEFR) and of delivering drugs used in the treatment of asthma_and comprising a peak flow meter and devices for delivering a drug used in the treatment of asthma

The device comprises a container in which the peak flow meter and the drug delivery devices are positioned and a mouthpiece suitable to be connected with the input of the peak flow meter, respectively with the output of an expansion chamber belonging to the drug delivery devices.

The drug delivery devices comprise a seat for a pressure cylinder containing the drug to be delivered, a nozzle for delivering the drug and the expansion chamber disposed between the nozzle and the mouthpiece, where the section of the expansion chamber is decreasing from the nozzle to its output, which is faced to the mouthpiece.

### Brief description of the Drawings

Now, the present invention will be clarified with reference to non-limiting examples of embodiment shown in the enclosed figures, wherein:
- Figure 1 is a side-view of the present invention, the internal structure of which is shown "by transparence";
- Figure 2 is a bottom-view of the present invention, performed according to A-A plane of Figure 1;
- Figure 3 is a vertical section of the present invention, performed according to B-B plane of Figure 1;
- Figure 4 is a horizontal section of the present invention, performed according to C-C plane of Figure 1;
- Figure 5 is a vertical section of the present invention, performed according to D-D plane of Figure 4;
- Figure 6 shows the mouthpiece 3 of Figure 5, separated from the context of said Figure;
- Figure 7 shows the part of the present invention which lays at the right side of the E-E plane of Figure 5, wherein the mouthpiece 3 is rotated of 180* with reference to the position of Figure 5;
- Figure 8 is a cross section of the mouthpiece 3 of Figure 7;
- Figure 9 is a cross section of the protective caps 4 indicated in Figure 7 by a broken line;
- Figure 10 is a vertical section, performed according to D-D plane of figure 4, of an embodiment of the device of Figure 1 comprising a device started by the breath of the patient;
- Figure 11 is a vertical section of a further embodiment of the present invention, comprising devices suitable for delivering a drug in the form of a powder.

In Figures enclosed, the corresponding elements will be identified by means of the same numeric references.

### Detailed Description of the Drawings

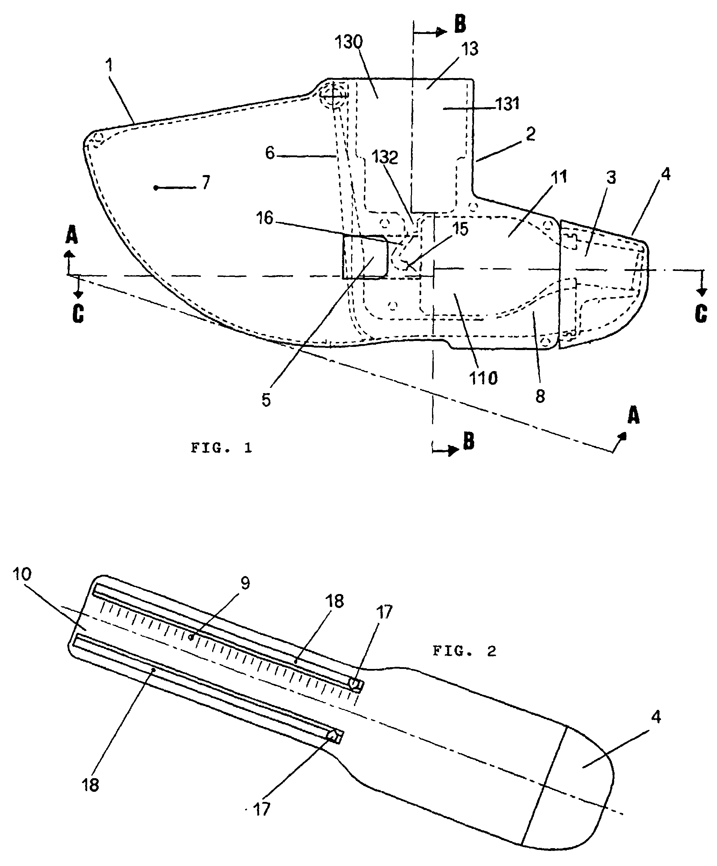

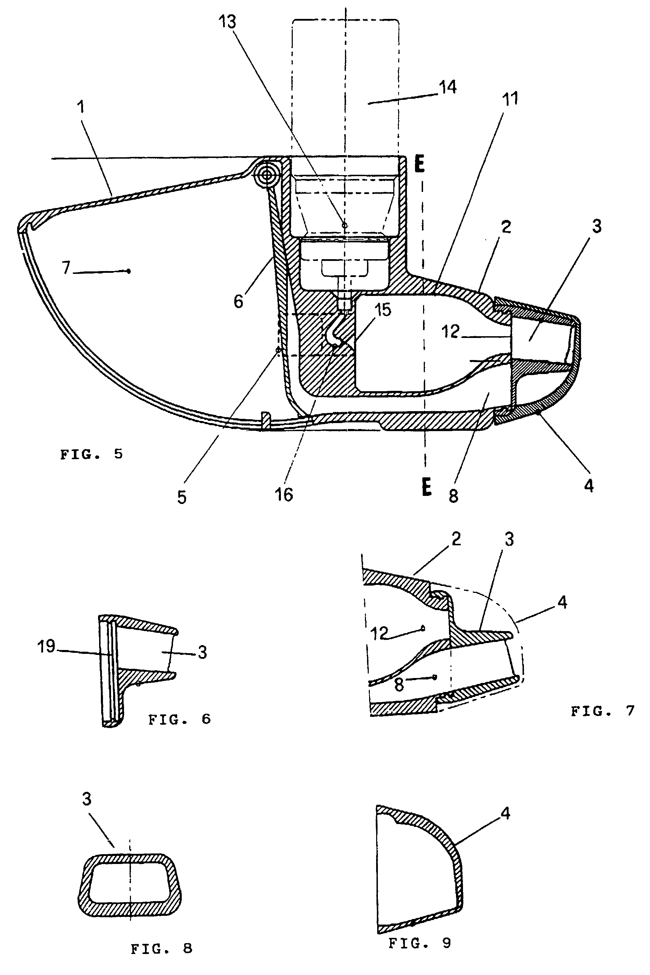

Figure 1 is a side-view of the present invention, the internal structure of which, shown "by transparence", is more clearly shown in the vertical section of Figure 5.

In Figure 1 the followings are indicated: peak flow meter 1, a delivery device 2 suitable for delivering the drug in the form of pressurized atomized liquid, the mouthpiece 3, covered by the protective cap 4 and a couple of openings 5 (one of which, only, is shown in Figure 5), provided on the side walls of the present invention to allow the air to be inspired together with the drug to enter the delivery device 2.

The meter 1 shown in Figure comprises a fin 6, hinged at one end and movable inside a cavity 7 (which has the form of a sector of circle) connected to the mouthpiece 3 by an inlet duct 8: the expired air flow rotates the fin 6 against the antagonist action of elastic means (not shown in Figure) and the greatest rotation amplitude of the fin 6, read on a suitable scale 9 (Figure 2) provided on the external face of the curvilinear wall 10 delimiting the cavity 7, allows to measure the PEFR.

Without going beyond the scope of the present invention, it's possible to substitute the meter 1 shown in Figures with another known PEFR meter.

The delivery device 2 comprises a seat 13, suitable for containing the pressure cylinder 14 containing the drug (Figure 5), connected by a duct 16 to a nozzle 15 from which the drug comes out (the nozzle 15 and the duct 16 are not disclosed herein, as they are known) and it differs from the traditional delivery devices in that they comprise an expansion chamber 11, disposed between the nozzle 15 and the mouthpiece 3, receiving the air to be mixed to the drug from outside through openings 5 provided on the side wall of the present invention.

By putting the expansion chamber 11 inside the device of the present invention, it has been possible to obtain a not very cumbersome device and a mixture of the drug with the air better than the one obtained by means of traditional delivery devices (the particles of the drug are smaller and dispersed more uniformly) and to reduce the speed with which the air-drug mixture gets to the patient, allowing the patient himself a better management of the drug during inspiration and consequently allowing an optimum penetration of the active molecules into the lower airways.

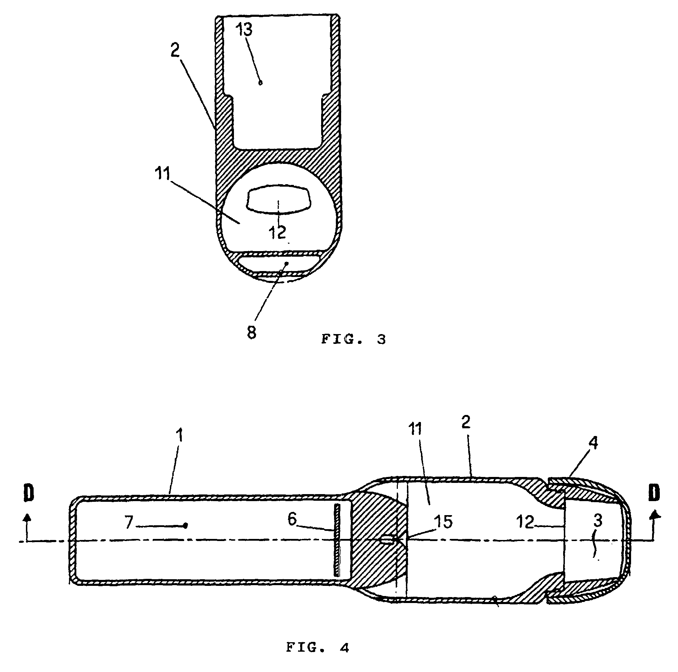

In Figures enclosed herein, it's possible to note that, in the embodiments herein shown, the expansion chamber 11 comprises a zone 110 having the form of a cylinder sectioned by a plane parallel to its longitudinal axis (Figure 3), connected to the output 12 having form and dimensions substantially corresponding to those of the mouthpiece 3; experimental verifications performed by the applicant allowed to obtain compact and efficient expansion chambers 11 having the following dimensions:
- cylinder diameter (zone 110): 25-30 mm (preferably 28 mm);
- maximum height of zone 110: 20-25 mm (preferably 23 mm);
- length of the zone 110: 17-23 mm (preferably 20 mm);
- total length of the expansion chamber 11 (from the bottom of the zone 110 to the output end 12): 30-40 mm (preferably 36 mm).

The present invention is also characterized in that it comprises a sole mouthpiece 3, which the patient can rotate to select each time the function he means to use (the meter 1 or the delivery device 2), by positioning the mouthpiece 3 connected with the output 12 of the expansion chamber 11 (Figures 1, 4, 5, 10 and 11) or connected with the inlet duct 8 of the meter 1 (Figure 7).

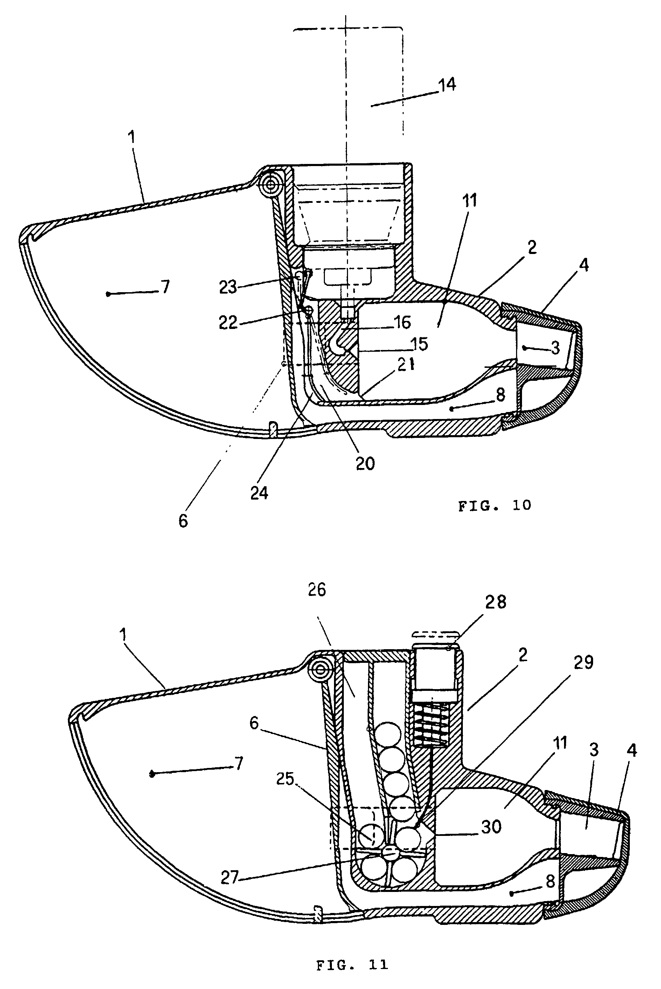

Many drugs used in the treatment of asthma and of other syndromes of the airways (cough, etc.) are currently on the market packaged on pressure cylinders MDI (Metered Dose Inhaler), one of which is schematically shown in Figures 5 and 10.

Such cylinders have a cylindrical base comprising the delivery valve of the drug, a cylindrical body and a surface connecting the base to an edge of the cylindrical body: at least in the most part of cylinders MDI the base and the delivery valve have the same dimensions, while the dimensions (diameter and length) of the cylindrical body and the height of the connecting surface (that is the distance between the base and the edge of the cylindrical body connected one another by said surface) are generally so different that each cylinder is generally sold together with a specific delivery device which isn't generally suitable for the intake of another drug (or of a drug packaged by a different firm).

The present invention is also characterized in that the delivery device 2 has a seat 13 having form and dimensions so that it is suitable for containing at least the most part of the pressure cylinders 14 currently on the market, allowing the patient to use the same delivery device 2 take different drugs or drugs manufactured by different firms, too.

For such purpose, the seat 13 has a first zone 130 having diameter not lower than the maximal diameter of the cylindrical body of cylinders 14 currently on the market and a second zone 131, underneath the first zone 130, having diameter not lower than the diameter of the base of cylinders 14 and having height not greater than the minimal height of the connecting surface of cylinders 14 currently on the market: the delivery valve of cylinder 14 disposed in the seat 13 is disposed in a flared hole 132 provided on the bottom of the second zone 131 and connected to the nozzle 15 by the duct 16.

Figure 2 shows a bottom-view of the present invention, performed according to A-A plane of Figure 1: in Figure it's possible to see the external face of the curvilinear wall 10 (delimiting the cavity 7 of the meter 1) on which the graduated scale 9 is provided, the indexes 17 (integral with the movable fin 6 of the meter 1) which move along the graduated scale 9 jutting out of the longitudinal slots 18 in the curvilinear wall 10, the lower wall of the duct 8 of the meter 1 and the external side of the protective cap 4.

Figure 3 shows a vertical section of the present invention, performed according to B-B plane of Figure 1: in Figure it's possible to see the seat 13 and the expansion chamber 11 provided in the main body of the delivery device 2, the output 12 of the expansion chamber 11 and the inlet duct 8 of the meter 1.

Figure 4 is a horizontal section of the present invention, performed according to C-C plane of Figure 1: in Figure it's possible to see the fin 6 and the cavity 7 of the meter 1, the nozzle 15 and the expansion chamber 11 comprising the output 12 of the delivery device and the mouthpiece 3 covered by the protective cap 4.

Figure 5 is a vertical section of the present invention, performed according to D-D plane of Figure 4: in Figure it's possible to see better the elements of the meter 1 and of the delivery device 2 which have already disclosed with reference to Figure 1 (comprising one of the openings 5, shown by a broken line as it's not visible, being covered by the main body comprising the nozzle 15) and the cylinder 14, containing the drug, placed in its own seat 13.

Figure 6 shows the mouthpiece 3 of Figure 5, separated by the context of the Figure to point out the jutting out ring 19 (on the internal surface of the zone of the mouthpiece 3 adjacent an end of the main body of the present invention) which, by engaging to a corresponding annular seat on the external surface of said end of the main body of the present invention, connects the mouthpiece 3 to the main body of the present invention so that it allows the patient to rotate the mouthpiece 3 to select each time the function (the meter 1 or the delivery device 2) he means to use.

Figure 7 shows the zone of the main body of the present invention which lays at the right side of E-E plane of Figure 5, wherein the mouthpiece 3 (rotated of 180° with reference to the position of Figure 5) is placed in connection with the inlet duct 8 of the meter 1.

Figure 8 is a cross-section of the mouthpiece 3 of Figure 7.

Figure 9 is a cross-section of the protective cap 4, shown in Figure 7 by a broken line.

Figure 10 is a vertical section, performed according to D-D plane of Figure 4, of an embodiment of the device of Figure 1 wherein the delivery device 2 comprises a device which stops the delivery of the drug from the cylinder 14 preventing its starting accidentally and/or before the patient is ready to take the drug, by starting the delivery of the drug by breath.

Such device consists of a lever, mobile in a hollow 20 provided behind the part comprising the duct 16 and the nozzle 15 and connected to the expansion chamber 11 by a passage 21, such lever being pivoted on a pivot 22 disposed in the hollow 20 and being kept on working position (indicated in Figure by a continuous line) by elastic means not shown in Figure.

On working position, the thickened end of the arm 23 of the lever engages against the base of the cylinder 14 keeping it up and therefore preventing the delivery valve of the cylinder itself from being started accidentally or in the wrong moment; in reply to the vacuum caused by the patient in the expansion chamber 11 when he starts to inspire to take the drug, the end of the arm 24 of the lever moves towards the expansion chamber 11, moving the lever itself to the position indicated in Figure 10 by a broken line: the rotation of the lever on the pivot 22 disengages the arm 23 from the base of the cylinder 14, releasing the cylinder itself and therefore allowing the patient to start the corresponding delivery valve to take the drug.

To semplify the drawings, the Figure doesn't show a pin (or similar device) which could be started from outside to stop the lever on working position.

Figure 11 shows a vertical section of a further embodiment of the present invention comprising delivery devices 2 suitable for delivering the drug in the form of a powder.

The drug is contained in capsules 25 inserted in a horizontal position in a loader 26 and moved ahead by a revolving device 27 started by the patient in a known manner.

To take the drug, the patient removes the protective cap 4 uncovering the mouthpiece 3, acts on the push-button 28 (having a return spring) connected to the blade 29 which rends the envelope of the capsule 25 disposed in connection with the nozzle 30 (preferably, but not necessarily of a different form from the form of the nozzle 15 of Figure 1) provided on the back wall of the expansion chamber 11 and starts to inspire, causing a vacuum in the expansion chamber 11, which attracts the powder contained in the capsule 25 rent by the blade 29 into the expansion chamber itself (through the nozzle 30).

In the expansion chamber 11 the powder gets mixed with the air which enters through the side openings 5 (Figure 1) and the patient takes the drug while he is inspiring, through the mouthpiece 3, the air-powder mixture which has formed in the expansion chamber 11.

Without going beyond the scope of the present invention, a technician can modify and improve the device for the treatment of asthmatic patients subject-matter of the present invention according to experience and to the natural progress of the technics.

## Claims

1. Device for the treatment of asthmatic patients, suitable for performing the double function of peak expiratory flow rate meter and of device for delivering drugs used in the treatment of asthma and comprising a peak flow meter (1) and devices (2) for delivering a drug used in the treatment of asthma, comprising a container in which the peak flow meter (1) and the drug delivery devices (2) are positioned and a mouthpiece (3) suitable to be connected with the input of the peak flow meter (1), respectively with the output (12) of an expansion chamber (11) belonging to the drug delivery devices (2); the drug delivery devices (2) comprise a seat (13) for a pressure cylinder (14) or for a loader (26) containing the drug to be delivered, **characterized in that** it comprises a nozzle (15, 30) for delivering said drug and an expansion chamber (11), disposed between the nozzle (15, 30) and the mouthpiece (3), the section of the expansion chamber (11) being decreasing from the nozzle (15, 30) to its output (12), which is faced to the mouthpiece (3).

2. Device according to claim 1, **characterized in that** the expansion chamber (11) receives from outside the air to be mixed to the drug through openings (5) provided on the side walls of the device.

3. Device according to claim 1, **characterized in that** the mouthpiece (3) is suitable to be rotated by the patient to be connected with an inlet duct (8) belonging to the peak flow meter (1), respectively with the output (12) of the expansion chamber (11) for delivering drug used in the treatment of asthma.

4. Device according to claim 3, **characterized in that** on the internal surface of the zone of the mouthpiece (3) connected to an end of the container a jutting out ring (19) is provided, suitable for engaging to a corresponding annular seat provided on the external surface of the end of the container, the rotation of the jutting out ring (19) in said annular seat allowing said the mouthpiece (3) to be connected with the inlet duct (8) of the peak flow meter (1), respectively with the output (12) of the expansion chamber (11).

5. Device according to claim 1, **characterized in that** the drug delivering devices (2) deliver the drug as a pressurised atomised liquid, said drug being contained in a pressure cylinder (14) to be positioned into the seat (13) of the drug delivery devices (2) and comprising a cylindrical base comprising the deliver valve of said drug, a cylindrical body and a surface connecting the cylindrical body to the base.

6. Device according to claim 5, **characterized in that** the seat (13) of the drug delivery devices (2) has such form and dimensions to be suitable for containing at least the most part of the pressure cylinders (14) available on the market.

7. Device according to claim 6, **characterized in that** the seat (13) comprises a first zone (130) having a diameter not lower than the maximum diameter of the cylindrical body of the pressure cylinders (14) and a second zone (131), underneath the first zone (130), having a diameter not lower than the diameter of the base of the pressure cylinders (14) and a height not greater than the minimum height of the connecting surface of the pressure cylinders (14), the delivery valve of the pressure cylinder (14) disposed in the seat (13) being disposed in a flared hole (132) provided at the bottom of the second zone (131) and connected to the nozzle (15) by a duct (16).

8. Device according to claim 7, **characterized in that** the drug delivery devices (2) also comprise a device suitable for stopping the delivery of the drug from the pressure cylinder (14) containing the drug, said device being stopped by the patient at the beginning of inspiration.

9. Device according to claim 8, **characterized in that** the device suitable for stopping the delivery of the drug consists of a lever, mobile in a hollow (20) provided in the drug delivery devices (2) for delivering the drug used in the treatment of asthma and connected to the expansion chamber (11) by a passage (21), said lever being pivoted and said pivot (22) being disposed in the hollow (20); **in that**, on working position, the end of a first arm (23) of the lever engages against the base of the cylinder (14) keeping it up and preventing the delivery valve of the cylinder (14) from being started; and **in that**, in reply to the vacuum caused by the patient in the expansion chamber (11) at the beginning of the inspiration performed to take the drug, the end of a second arm (24) of the lever moves towards the expansion chamber (11), rotating the lever around the pivot (22), disengaging the end of the first arm (23) of the lever from the base of the cylinder (14), releasing the cylinder (14) and allowing the delivery valve of the cylinder (14) to be started.

10. Device according to claim 9, **characterized in that** the lever is kept on working position by elastic means.

11. Device according to claim 1, **characterized in that** the drug delivery devices (2) deliver said drug in the form of a powder.

12. Device according to claim 11, **characterised in that** the drug delivery devices (2) comprise a loader (26), a plurality of capsules (25) containing the drug in the form of a powder inserted in a horizontal position in the loader (26) and moved ahead by a revolving device (27) started by the patient and a push-button (28) activated by the patient and comprising a return spring, connected to a blade (29) suitable for rending the envelope of the capsule (25) disposed connected with a nozzle (30) provided on the back wall of the expansion chamber (11); and **in that**, in reply to the vacuum caused by the patient in the expansion chamber (11) during the inspiration performed to take the drug, said drug in the form of a powder contained in the capsule (25) rent by the blade (29) is attracted, through the nozzle (30), to the expansion chamber (11), wherein it gets mixed with the air coming from outside through the openings (5) provided on the side walls of the device to be taken by the patient.

## Patentansprüche

1. Vorrichtung zur Behandlung von Asthma-Patienten, welche zur Ausführung der Doppelfunktion eines Spitzenwert-Volumenstrommessers für die Ausatmungshöchstwerte und einer Vorrichtung für die Abgabe von Arzneimitteln, wie sie bei der Behandlung von Asthma Verwendung finden, geeignet ist und welche einen Spitzenwert-Volumenstrommesser (1) und Vorrichtungen (2) für die Abgabe eines bei der Behandlung von Asthma eingesetzten Arzneimittels, einen Behälter, in welchem der Spltzenwert-Volumenstrommesser (1) und die die Arzneimittel abgebenden Vorrichtungen (2) untergebracht sind, und ein Mundstück (3) umfasst, welches dazu geeignet ist, mit der Eintrittsöffnung des Spitzenwert-Volumenstrommessers (1) beziehungsweise mit der Austrittsöffnung (12) einer Expansionskammer (11), welche zu den die Arzneimittel abgebenden Vorrichtungen (2) gehört, verbunden zu werden; wobei die die Arzneimittel abgebenden Vorrichtungen (2) einen Sitz (13) für eine Druckgasflasche (14) oder für ein Dosiergerät (26) aufweisen, welche das abzugebende Arzneimittel enthalten, **dadurch gekennzeichnet, dass** die besagte Vorrichtung eine Düse (15, 30) für die Abgabe des besagten Arzneimittels und eine zwischen der Düse (15, 30) und dem Mundstück (3) angeordnete Expansionskammer (11) aufweist, wobei der Querschnitt der Expansionskammer (11) von der Düse (15, 30) bis zu ihrer Austrittsöffnung (12), welcher dem Mundstück (3) zugewandt ist, kleiner wird.

2. Vorrichtung nach Anspruch 1, **dadurch gekennzeichnet, dass** die Expansionskammer (11) die Luft, die mit dem Arzneimittel vermischt werden soll, von außen durch die in den Seitenwänden der Vorrichtung angebrachten Öffnungen (5) erhält.

3. Vorrichtung nach Anspruch 1, **dadurch gekennzeichnet, dass** das Mundstück (3) dazu geeignet ist, vom Patienten gedreht zu werden, so dass es mit einem zum Spitzenwert-Volumenstrommesser (1) gehörenden Einlasskanal (8) beziehungsweise mit der Austrittsöffnung (12) der Expansionskammer (11) für die Abgabe des für die Behandlung von Asthma verwendeten Arzneimittels verbunden werden kann.

4. Vorrichtung nach Anspruch 3, **dadurch gekennzeichnet, dass** auf der Innenseite des Bereiches des Mundstückes (3), welcher mit einem Ende des Behälters verbunden ist, ein vorstehender Ring (19) vorhanden ist, welcher dazu geeignet ist, in einen passenden ringförmigen Sitz, welcher auf der Außenseite des Endbereichs des Behälters angeordnet ist, einzurasten; wobei das Drehen des vorspringenden Außenringes (19) in dem besagten ringförmigen Sitz ermöglicht, dass das besagte Mundstück (3) mit dem Einlasskanal (8) des Spltzenwert-Volumenstrommessers (1) beziehungsweise mit der Austrittsöffnung (12) der Expansionskammer (11) verbunden wird.

5. Vorrichtung nach Anspruch 1, **dadurch gekennzeichnet, dass** die das Arzneimittel abgebenden Vorrichtungen (2) das Arzneimittel als eine unter Druck stehende, fein zerstäubte Flüssigkeit abgeben, wobei das besagte Arzneimittel in einer Druckgasflasche (14) enthalten ist, welche in den Sitz (13) der das Arzneimittel abgebenden Vorrichtungen (2) einzusetzen ist und welche ein zylinderförmiges Basisteil, welches das Austrittsventil für das besagte Arzneimittel enthält, einen zylinderförmigen Körper und eine Fläche, die den zylinderförmigen Körper mit dem Basisteil verbindet, aufweist.

6. Vorrichtung nach Anspruch 5, **dadurch gekennzeichnet, dass** der Sitz (13) der das Arzneimittel abgebenden Vorrichtungen (2) eine solche Form und solche Abmessungen hat, die für die Aufnahme von zumindest des größten Teiles der auf dem Markt erhältlichen Druckgasflaschen (14) geeignet sind.

7. Vorrichtung nach Anspruch 6, **dadurch gekennzeichnet, dass** der Sitz (13) einen ersten Bereich (130), dessen Durchmesser nicht kleiner als der Maximaldurchmesser des zylindrischen Körpers (14) der Druckgasflaschen (14) ist, und unterhalb des ersten Bereichs einen zweiten Bereich (131) aufweist, dessen Durchmesser nicht kleiner als der Durchmesser des Basisteils der Druckgasflaschen (14) und dessen Höhe nicht größer als die Minimalhöhe der Verbindungsfläche der Druckgasflaschen (14) sind, wobei sich das in dem Sitz (13) angeordnete Austrittsventil der Druckgasflasche (14) in einem aufgeweiteten Loch (132) befindet, welches im Boden des zweiten Bereichs (131) vorhanden ist und mit der Düse (15) über einen Kanal (16) verbunden ist.

8. Vorrichtung nach Anspruch 7, **dadurch gekennzeichnet, dass** die das Arzneimittel abgebenden Vorrichtungen (2) auch eine Vorrichtung enthalten, welche dazu geeignet ist, die Abgabe des Arzneimittels aus der das Arzneimittel enthaltenden Druckgasflasche (14) zu unterbrechen, wobei die besagte Vorrichtung vom Patienten beim Beginn des Einatmens gestoppt wird.

9. Vorrichtung nach Anspruch 8, **dadurch gekennzeichnet,**
**dass** die Vorrichtung, die dafür geeignet ist, die Zufuhr des Arzneimittels zu unterbrechen, aus einem Hebel besteht, welcher in einem Hohlraum (20) beweglich ist, der in den die Arzneimittel abgebenden Vorrichtungen (2) für die Abgabe des bei der Behandlung von Asthma verwendeten Arzneimittels vorhanden und der mittels eines Durchganges (21) mit der Expansionskammer (11) verbunden ist, wobei der besagte Hebel drehbar gelagert ist und das *besagte* Drehgelenk (22) in dem Hohlraum (20) angeordnet ist;
**dass** in der Betriebsstellung das Ende eines ersten Arms (23) des Hebels gegen das Basisteil der Gasflasche (14) drückt, ihn angehoben hält damit die Inbetriebsetzung des Austrittsventils verhindert;
und **dass** als Reaktion auf den Unterdruck, welcher in der Expansionskammer (11) vom Patienten bei Beginn des Einatmens zum Zweck der Aufnahme des Arzneimittels hervorgerufen wird, sich das Ende eines zweiten Arms (24) des Hebels in Richtung auf die Expansionskammer (11) bewegt, wobei sich der Hebel um das Drehgelenk (22) dreht, so dass sich das Ende des ersten Armes (23) des Hebels vom Basisteil der Gasflasche (14) löst, wodurch die Gasflasche (14) freigegeben und dem Austrittsventil der Gasflasche (14) die Möglichkeit zur Betätigung gegeben wird.

10. Vorrichtung nach Anspruch 9, **dadurch gekennzeichnet, dass** der Hebel durch elastische Mittel in Betriebsstellung gehalten wird.

11. Vorrichtung nach Anspruch 1, **dadurch gekennzeichnet, dass** die das Arzneimittel abgebenden Vorrichtungen (2) besagtes Arzneimittel in Form eines Pulvers abgeben.

12. Vorrichtung nach Anspruch 11, **dadurch gekennzeichnet,**
**dass** die das Arzneimittel abgebenden Vorrichtungen (2) enthalten: ein Dosiergerät (26), eine Anzahl von Kapseln (25), welche das Arzneimittel in der Form eines Pulvers enthalten und in einer horizontalen Lage in das Dosiergerät (26) eingelegt und mittels einer vom Patienten gestarteten Drehvorrichtung (27) nach vorn bewegt werden, und einen vom Patienten betätigten Taster (28), der eine Rückholfeder enthält, die mit einer Schneide (29) verbunden ist, welche zum Aufbrechen der Umhüllung derjenigen Kapsel (25) geeignet ist, die so angeordnet ist, dass sie mit einer auf der Rückwand der Expansionskammer (11) angeordneten Düse (30) in Verbindung steht,
und **dass** als Reaktion auf den Unterdruck, der in der Expansionskammer (11) vom Patienten während des Einatmens zum Zweck der Aufnahme des Arzneimittels hervorgerufen wird, das besagte Arzneimittel in Form eines Pulvers, das in der von der Schneide (29) aufgebrochenen Kapsel (25) enthalten ist, durch die Düse (30) angesaugt und zur Expansionskammer (11) gesaugt wird, in welcher es mit der Luft vermischt wird, die von außen durch die in den Seitenwänden der Vorrichtung befindlichen Öffnungen (5) strömt, so dass diese vom Patienten aufgenommen werden kann.

## Revendications

1. Dispositif de traitement de patients asthmatiques, adapté pour effectuer la double fonetion de mesure du débit d'expiration de pointe et de dispositif destiné à distribuer des médicaments utilisés dans le traitement de l'asthme et comportant un débitmètre de pointe (1) et des dispositifs (2) pour distribuer un médicament utilisé dans le traitement de l'asthme, comportant un conteneur dans lequel le débitmètre de pointe (1) et les dispositifs de distribution de médicament (2) sont positionnés et une embouchure (3) adaptée pour être reliée à l'entrée du débitmétre de pointe (1), respectivement à la sortie (12) d'une chambre d'expanaion (11) appartenant aux dispositifs de distribution de médicament (2), les dispositifs de distribution de médicament (2) comportant un siège (13) pour un cylindre sous pression (14) ou pour un chargeur (26) contenant le médicament à distribuer, **caractérisé en ce qu'**il comporte une buse (15), 30) destinée à distribuer ledit médicament et une chambre d'expansion (11), disposée entre la buse (15, 30) et l'embouchure (3), la section de la chambre d'expansion (11) diminuant à partir de la buse (15, 30) vers sa sortie (12), qui est dirigée vers l'embouchure (3).

2. Dispositif selon la revendication 1, **caractérisé en ce que** la chambre d'expansion (11) reçoit, à partir de l'extérieur, de l'air à mélanger au médicament, à travers des ouvertures (5) agencées dans les parois latérales du dispositif.

3. Dispositif selon la revendication 1, **caractérisé en ce que** l'embouchure (3) est adaptée pour être mise en rotation par le patient pour être reliée à un conduit d'entrée (8) appartenant au débitmètre de pointe (1), et respectivement à la sortie (12) de la chambre d'expansion (11) pour distribuer un médicament utilisé dans le traitement de l'asthme.

4. Dispositif selon la revendication 3, **caractérisé en ce que** sur la surface intérieure de la zone de l'embouchure (3) reliée à une extrémité du conteneur, est agencé un anneau de sortie (19), adapté pour coopérer avec un siège annulaire correspondant agencé sur la surface extérieure de l'extrémité du conteneur, la rotation de l'anneau de sortie (19) dans ledit siège annulaire permettant que ladite embouchure (3) soit reliée au conduit d'entrée (8) du débitmètre de pointe (1), et respectivement à la sortie (12) de la chambre d'expansion (11).

5. Dispositif selon la revendication 1, **caractérisé en ce que** les dispositifs de distribution de médicament (2) distribuent le médicament sous forme d'un liquide atomisé sous pression, ledit médicament étant contenu dans un cylindre sous pression (14) destiné à être positionné dans le siège (13) des dispositifs de distribution de médicament (2) et comportant une base cylindrique comportant la soupape de distribution de médicament, un corps cylindrique et une surface reliant le corps cylindrique à la base.

6. Dispositif selon la revendication 5, **caractérisé en ce que** le siège (13) des dispositifs de distribution de médicament (2) a une forme et des diménsions telles qu'il est adapté pour contenir au moins la majeure partie des cylindres sous pression (14) disponibles sur le marché.

7. Dispositif selon la revendication 6, **caractérisé en ce que** le siège (13) comporte une première zone (130) ayant un diamètre qui n'est pas inférieur au diamètre maximum du corps cylindrique des cylindres sous pression (14) et une seconde zone (131), située en dessous de la première zone (130), ayant un diamètre qui n'est pas inférieur au diamètre de la base des cylindres sous pression (14) et une hauteur qui n'est pas plus grande que la hauteur minimum de la surface de liaison des cylindres sous pression (14), la soupape de distribution du cylindre sous pression (14) qui est disposé dans le siège (13) étant disposée dans un trou évasé (132) agencé au niveau de la partie inférieure de la seconde zone (131) et relié à la busé (15) par un conduit (16).

8. Dispositif selon la revendication 7, **caractérisé en ce que** les dispositifs de distribution de médicament (2) comportent aussi un dispositif adapté pour arrêter la distribution du médicament à partir du cylindre sous pression (14) contenant le médicament, ledit dispositif étant arrêté par le patient au début d'une inspiration.

9. Dispositif selon la revendication 8, **caractérisé en ce que** le dispositif adapté pour arrêter la distribution du médicament est constitué d'un levier, mobile dans une partie creuse (20) agencée dans le dispositif de distribution du médicament (2) pour distribuer des médicaments utilisés dans le traitement de l'asthme et reliée à la chambre d'expansion (11) par un passage (21), ledit levier étant pivoté et ledit pivot (22) étant disposé dans la partie creuse (20), **en ce que**, dans une position active, l'extrémité d'un premier bras (23) du levier vient en contact contre la base du cylindre (14) le maintenant vers le haut et empêchant que la soupape de distribution du cylindre (14) ne soit démarrée, et **en ce que**, en réponse au vide provoqué par le patient dans la chambre d'expansion (11) au début de l'inspiration effectuée pour prendre le médicament, l'extrémité d'un second bras (24) du levier se déplace en direction de la chambre d'expansion (11), mettant en rotation le levier autour du pivot (22), libérant l'extrémité du premier bras (23) du levier de la base du cylindre (14), libérant le cylindre (14) et permettant de démarrer la soupape de distribution du cylindre (14).

10. Dispositif selon la revendication 9, **caractérisé en ce que** le levier est maintenu dans la position active par des moyens élastiques.

11. Dispositif selon la revendication 1, **caractérisé en ce que** les dispositifs de distribution de médicament (2) distribuent ledit médicament sous la forme d'une poudre.

12. Dispositif selon la revendication 11, **caractérisé en ce que** le dispositif de distribution de médicament (2) comporte un chargeur (26), une pluralité de capsules (25) contenant le médicament sous la forme d'une poudre insérées dans une position horizontale dans le chargeur (26) et déplacées vers l'avant par un dispositif tournant (27) démarré par le patient et un bouton poussoir (28) activé par le patient et comportant un ressort de rappel, relié à une lame (29) adaptée pour déchirer l'enveloppe de la capsule (25) disposée reliée à une buse (30) agencée sur la paroi arrière de la chambre d'expansion (11), et **en ce qu'**en réponse au vide provoqué par le patient dans la chambre d'expansion (11) pendent l'inspiration effectuée pour prendre le médicament, ledit médicament sous la forme d'une poudre contenue dans la capsule (25) déchirée par la lame (29) est attiré, à travers la buse (30), vers la chambre d'expansion (11), dans laquelle il est mélangé à l'air provenant de l'extérieur à travers les ouvertures (5) agencées dans les parois latérales du dispositif pour être pris par le patient.
